# EUROPÄISCHE PATENTSCHRIFT

(11) **EP 1 022 004 B1**
(45) Veröffentlichungstag und Bekanntmachung des Hinweises auf die Patenterteilung: **10.12.2003**
(21) Anmeldenummer: 00101157.6
(22) Anmeldetag: 21.01.2000
(51) Int. Cl.: A61F 13/20

(54) **Vorrichtung zum Pressen eines Tampons, insbesondere für die Frauenhygiene**
Device for the pressing of tampons, particulary for women's hygiene
Dispositif pour presser des tampons, en particulier pour l'hygiène féminine

(30) Priorität: 23.01.1999 DE 19902643
(43) Veröffentlichungstag der Anmeldung: 26.07.2000
(73) Patentinhaber: Ruggli Projects AG, 6332 Hagendorn Cham (CH)
(72) Erfinder: Kuhn, Kurt, 8175 Windlach (CH)
(74) Vertreter: Christophersen, Ulrich Rudolf

(56) Entgegenhaltungen:
- EP-A- 0 639 363
- DE-A- 4 304 505

## Beschreibung

Die Erfindung betrifft eine Vorrichtung zum Pressen eines Tampons, insbesondere für die Frauenhygiene, mit über den Umfang eines zu pressenden Tamponrohlings gleichmäßig verteilt angeordneten Preßwerkzeugen, die radial zur Längsrichtung des Tamponrohlings verfahrbar sind und mit denen die gesamte Umfangsfläche des Tamponrohlings mit radialem Druck beaufschlagbar ist, wobei die Preßwerkzeuge jeweils eine Preßfläche aufweisen, die sich aus der Stimfläche einer Preßschulter zur Bildung von verhältnismäßig weichen Längsrippen hoher Absorptionsfähigkeit und einer im Vergleich zur Preßschulter in radialer Richtung vorstehenden Preßschneide zur Bildung eines vergleichsweise verdichteten Tamponkems hoher Knickfestigkeit zusammensetzt.

Bekannte Tampons bestehen üblicherweise aus einem Faservlies, das zunächst zu einem Tamponrohling gewickelt und anschließend auf die gewünschte Endform des Tampons gepreßt wird. Die Anforderungen, die an solche Tampons gestellt werden, sind vornehmlich eine hohe Formsteifigkeit und Saugleistung. Während die Formsteifigkeit von der durch die Höhe der Verdichtung des Faservlies beim Pressen erzeugten Knickfestigkeit abhängt, wird die Saugleistung durch die von dem Tampon bewirkte Sauggeschwindigkeit und Absorptionsfähigkeit bestimmt. Letzere lassen sich dann in hohem Maße erreichen, wenn das Faservlies eine hohe Kapillarität aufweist und demzufolge von einer gering verdichteten, weichen Faserstruktur ist.

Eine diesen gegenläufigen Anforderungen Rechnung tragende, gattungsgemäße Vorrichtung ist aus der DE 43 04 505 A1 bekannt. Mittels der Preßschneiden der Preßwerkzeuge dieser Vorrichtung lassen sich diskrete Bereiche der Umfangsfläche eines Tamponrohlings zu einem verhältnismäßig hoch verdichteten zentralen Faserkem hoher Knickfestigkeit pressen. Die zwischen den Preßschneiden befindlichen Bereiche der Umfangsfläche des Tamponrohlings werden hingegen von den im Vergleich zu den Preßschneiden in radialer Richtung zurückstehenden Preßschultern mit radialem Druck beaufschlagt und erfahren dadurch eine geringere Verformung mit der Folge, daß die dem Tamponrohling immanente grobe Faserstruktur weitgehend erhalten bleibt. Die auf diese Weise gebildeten verhältnismäßig weichen Längsrippen hoher Absorptionsfähigkeit des Tampons lassen sich dann in einem nachgeschalteten Formwerkzeug zu einer nahezu geschlossenen Oberfläche verformen, ohne dabei die Faserstrukturen im Kern- und Außenbereich des Tampons wesentlich zu verändern.

Nachteilig bei der zuvor beschriebenen, gattungsgemäßen Vorrichtung ist, daß eine Anpassung an im Durchmesser unterschiedliche Tampons mit einem verhältnismäßig hohen Aufwand verbunden ist. Dies ist darauf zurückzuführen, daß die aus der DE 43 04 505 A1 bekannte Vorrichtung im Vergleich zu konventionellen Tamponpressen nicht mehr zwei oder mehr Gruppen mit unterschiedlichen Preßbacken, sondern nur noch Preßwerkzeuge eines einzigen Typs, an denen jeweils eine Preßschneide und eine Preßschulter ausgebildet ist, aufweist. Die sich hieraus ergebende Folge ist, daß das Verhältnis der Durchmesser von vergleichsweise hoch verdichtetem Tamponkem und durch die Preßschultern geformtem Umfang stets konstant ist. Die Anpassung dieses Verhältnisses an einen Tampon mit anderem Durchmesser bedingt daher einen wirtschaftlich nicht befriedigenden Austausch sämtlicher Preßwerkzeuge.

Der Erfindung liegt die **Aufgabe** zugrunde, eine Vorrichtung zum Pressen eines Tampons dahingehend weiterzubilden, daß sich auf einfache und kostengünstige Weise eine Anpassung an unterschiedlich große Tampons erzielen läßt.

Diese Aufgabe ist bei einer Vorrichtung mit den eingangs genannten Merkmalen erfindungsgemäß dadurch **gelöst**, daß die Preßschulter und die Preßschneide eines jeden Preßwerkzeugs relativ zueinander verstellbar sind.

Eine solchermaßen ausgebildete Vorrichtung ermöglicht auf einfache und kostengünstige Weise eine Anpassung an unterschiedlich groß zu pressende Tampons. Ursächlich hierfür ist, daß das Verhältnis der Durchmesser von Tamponkem und -umfang durch die relative Verstellbarkeit von Preßschulter und Preßschneide auf den jeweils angestrebten Tampondurchmesser eingestellt werden kann. Besonders zum Tragen kommt hierbei auch, daß das Materialverhältnis von verdichtetem Tamponkem und den diesen umgebenden, verhältnismäßig weichen Längsrippen mit gröberer Faserstruktur auf einfache Weise variiert werden kann. Ein kosten- und zeitaufwendiger Austausch der Preßwerkzeuge bei einem Wechsel auf einen anderen Tampondurchmesser läßt sich damit vermeiden.

In einer bevorzugten Ausgestaltung der Erfindung ist die Preßschulter an einer Seitenfläche der Preßschneide in radialer Richtung verschiebbar angeordnet, um eine in konstruktiver Hinsicht einfache Gestaltung zu erreichen. Von besonderem Vorteil hierbei ist es, die Seitenfläche der Preßschneide als Führungsbahn für die Preßschulter auszubilden, wobei die Führungsbahn vorzugsweise eine flache Querschnittsform aufweist. Auf diese Weise ist ein schnell durchzuführendes, in radialer Richtung geradliniges Verstellen der Preßschulter in Bezug auf die Preßschneide sichergestellt. Die Führungsbahn kann dabei in an sich bekannter Weise als flache Gleitführung oder mit einer profilierten Querschnittsform, etwa in Form eines oder mehrerer, in radialer Richtung parallelen V- oder Schwalbenschwanzprofilen ausgebildet sein.

Gemäß einer bevorzugten Weiterbildung der Erfindung ist die Preßschulter und/oder die Preßschneide mit wenigstens einem Langloch für eine Schraubenverbindung versehen, mit der die Preßschulter und die Preßschneide relativ zueinander verstellbar und lösbar miteinander verbindbar sind. Das Vorsehen eines Langloches im Zusammenhang mit einer Schraubenverbindung zum lösbaren Verbinden von Preßschulter und -schneide bietet den Vorteil einer zuverlässigen und in ihrer Größe beschränkbaren Verstellbarkeit bei einfacher und kostengünstiger Fertigung. Indem die Preßschulter und die Preßschneide eines Preßwerkzeugs lösbar miteinander verbindbar sind, ist zudem ein beispielsweise verschleißbedingter, getrennter Austausch von Preßschulter und/oder -schneide gewährleistet. In jenen Fällen, in denen aus ergonomischen und haptischen Gründen ein Tampon mit einem verdickten Ende, beispielsweise zum Ausbilden einer rückseitigen Fingermulde angestrebt wird, ist es zweckmäßig, wenigstens zwei unterschiedlich orientierte Langlöcher vorzusehen, so daß sich die Preßschulter schräg zur Preßschneide anordnen läßt. Die schräge Anordnung der Preßschulter hat zur Folge, daß der Tampon etwa im Bereich seines hinteren Endes geringer verformt wird, um das vorgenannte verdickte Tamponende zu bilden. Die aus der DE 43 04 505 A1 zu diesem Zweck bekannten Preßschultern mit einem stufig abgesetzten oder schräg verlaufenden Abschnitt der Stimfläche und der damit in fertigungstechnischer und kostenmäßiger Hinsicht verbundene Aufwand ist damit hinfällig.

Gemäß einem weiteren Merkmal der Erfindung ist zumindest ein Teil der der Preßschneide abgewandten Seitenfläche der Preßschulter als Anlagefläche für das benachbart angrenzende Preßwerkzeug ausgebildet. Die Anlagefläche kann dabei derart ausgestaltet sein, daß die Preßwerkzeuge im geschlossenen Zustand zwar aneinander stoßen, im Bereich der Preßfläche aber durch einen jeweiligen Zwischenraum in Umfangsrichtung voneinander separiert sind. Der Zwischenraum verhindert dabei, daß beim Schließen der Preßwerkzeuge Teile des Faservlies zwischen diesen eingeklemmt werden, wodurch die Struktur und damit die Kapillarität des Faservlies beeinträchtigt würde. Im Bereich des Aneinanderstoßens zweier jeweils benachbarter Preßwerkzeuge kann die Anlagefläche vorteilhafterweise als annähernd linienförmige Kontaktfläche ausgebildet sein, die verhindert, daß Faservlies außerhalb der Preßfläche gelangt.

In Weiterbildung der Erfindung wird ferner vorgeschlagen, die Preßschulter mit einer im wesentlichen konkaven Stirnfläche zu versehen, um eine weitgehende Anpassung des gepreßten Vorformlings an die Endform des Tampons zu erreichen. Von Vorteil ist ferner, die Preßschneide verhältnismäßig schmal und vorzugsweise an ihrem in radialer Richtung innen liegenden Ende sich konisch verjüngend auszubilden. Auf diese Weise ist sichergestellt, daß ausschließlich schmale Bereiche der Umfangsfläche zu dem vergleichsweise hoch verdichteten Tamponkem gepreßt werden, während der überwiegende Teil der Umfangsfläche des Vorformlings zur Bildung von verhältnismäßig weichen Längsrippen hoher Absorptionsfähigkeit dient. Eine konische Verjüngung des in radialer Richtung innen liegenden Endes der Preßschneide trägt ferner dazu bei, daß sich die Preßschneiden beim Öffnen der Preßwerkzeuge aus dem gepreßten Vorformling zuverlässig herausfahren lassen, ohne die gebildete Faserstruktur, etwa durch an den Preßschneiden anhaftendes Faservlies, im Nachhinein zu verändern.

Schließlich wird vorgeschlagen, daß die Preßwerkzeuge in radialer Richtung konzentrisch verfahrbar sind, um den Vorformling in einem Arbeitsschritt gleichmäßig zu pressen.

Einzelheiten und weitere Vorteile des Gegenstandes der Erfindung ergeben sich aus der nachfolgenden Beschreibung eines bevorzugten Ausführungsbeispieles. In den zugehörigen Zeichnungen zeigen im einzelnen:
- Fig. 1: eine schematische Ansicht einer Vorrichtung zum Pressen eines Tampons mit Presswerkzeugen in der geöffneten Stellung;
- Fig. 1a: eine schematische Ansicht der Vorrichtung gemäß Fig. 1 mit Preßwerkzeugen in der geschlossenen Stellung;
- Fig.1b: eine schematische Ansicht der Vorrichtung gemäß Fig. 1 mit Preßwerkzeugen in einer teilgeschlossenen Stellung und
- Fig. 2: eine teilgeschnittene Darstellung eines Preßwerkzeugs.

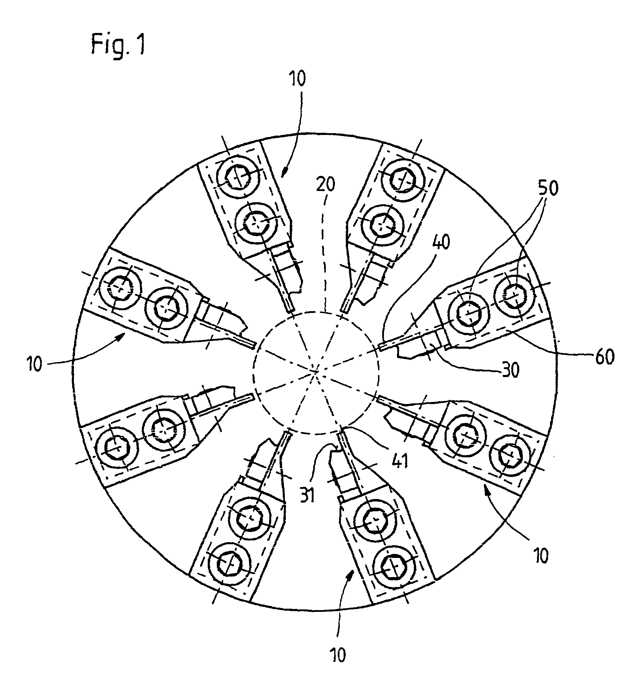

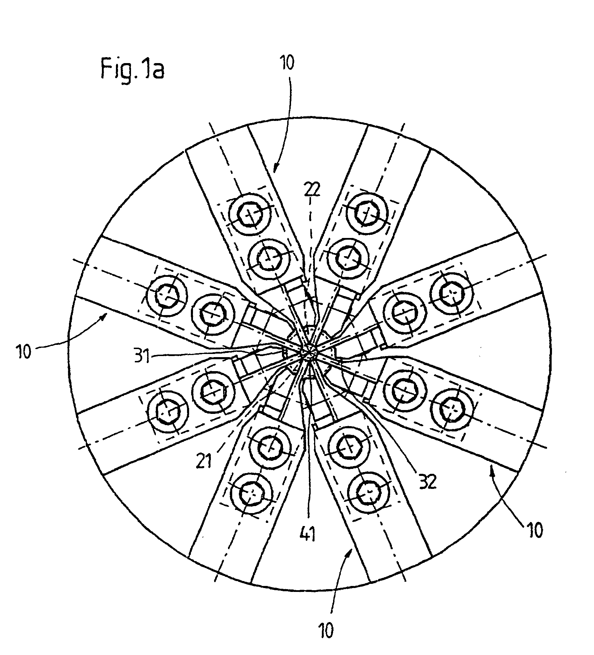

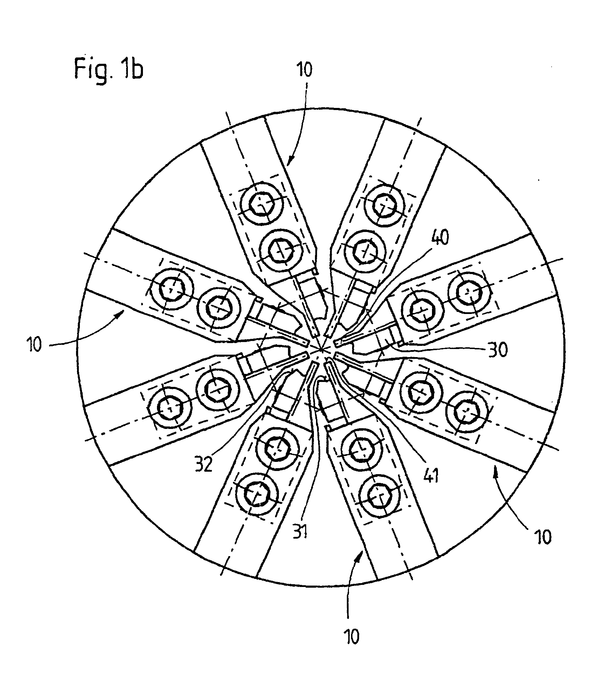

Die in den Figuren 1 bis 1b dargestellte Vorrichtung zum Pressen eines Tampons weist acht gleich ausgebildete Preßwerkzeuge 10 auf, die gleichmäßig verteilt über den Umfang eines zu pressenden Tamponrohlings 20 angeordnet sind. Die Preßwerkzeuge 10 setzen sich jeweils aus einer Preßschulter 30 und einer Preßschneide 40 zusammen und sind radial zur Längsrichtung des Tamponrohlings konzentrisch verfahrbar. Zu diesem Zweck sind die Preßwerkzeuge mittels Schraubenverbindungen 50 mit mechanisch betätigten Schieberführungen 60 verbunden. Die Befestigung mittels Schraubenverbindungen stellt dabei sicher, daß die Preßwerkzeuge 10 etwa bei auftretendem Verschleiß, auswechselbar sind.

Die Preßschneiden 40 sind verhältnismäßig schmal und an ihren in radialer Richtung innen liegenden Enden mit einem konstanten Querschnitt ausgebildet. Dies hat zur Folge, daß die Preßschneiden 40 eine nahezu ebene und in Längsrichtung des Tamponrohlings 20 betrachtet rechteckige Stirnfläche 41 aufweisen.

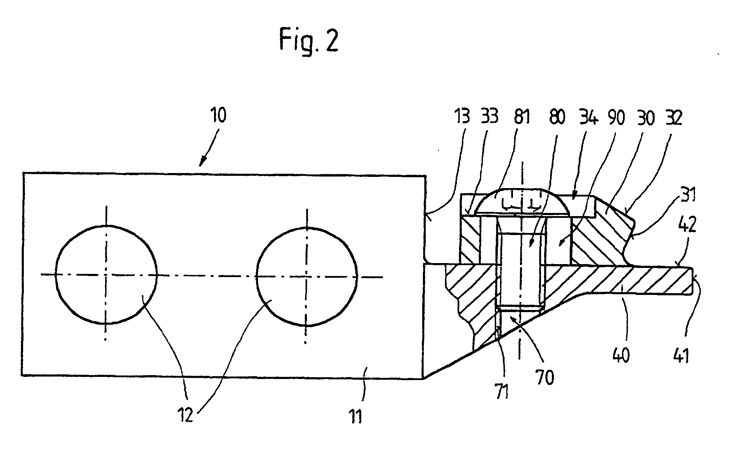

Die Preßschulter 30 und die Preßschneiden 40 eines jeden Preßwerkzeugs 10 sind relativ zueinander verstellbar. Die Preßschulter 30 ist hierzu an einer Seitenfläche 42 der Preßschneide 40 verschiebbar angeordnet, wie insbesondere Fig. 2 zu entnehmen ist. Die als flache Gleitführung ausgebildete Seitenfläche 42 weist zu diesem Zweck mehrere senkrecht zur Längsrichtung des Tamponrohlings 20 angeordnete Bohrungen 70 auf, die mit einem Innengewinde 71 zum Befestigen von Schrauben 80 versehen sind. Die Schrauben 80 werden in einem in den Preßschultern 30 ausgebildeten Langloch 90 geführt, dessen Längserstreckung den maximalen Verstellweg der Preßschulter 30 bezüglich den Preßschneiden 40 vorgibt. Um die Preßschulter 30 auf der Seitenfläche 42 der Preßschneide 40 zu fixieren, ist auf der der Preßschneide 40 abgewandten Seite der Preßschulter 30 eine Klemmfläche 33 für den Kopf 81 der Schraube 80 vorgesehen, die um etwa die Höhe des Kopfes 81 in einer Ausnehmung 34 der Preßschulter 30 versenkt ist.

Fig. 2 läßt ferner erkennen, daß die Preßschulter 30 eine zur radialen Richtung schräg verlaufende Anlagefläche 32 aufweist, die sich zwischen der Ausnehmung 34 und der annähernd konkav ausgebildeten Stirnfläche 31 der Preßschulter 30 erstreckt. Die Stirnfläche 31 der Preßschulter 30 bildet zusammen mit der gegenüber dieser in radialer Richtung vorstehenden Stirnfläche 41 der Preßschneide 40 die Preßfläche eines Preßwerkzeugs 10, die ein Segment der Umfangsfläche des zu pressenden Tamponrohlings 20 mit einem radialen Druck beaufschlagt. Am in radialer Richtung äußeren Ende der Preßschneide 40 ist ein Befestigungsabschnitt 11 vorgesehen, der Bohrungen 12 zum Befestigen an der Schieberführung 60 aufweist. Die Preßschneide 40 ist an ihrer der Preßschulter 30 abgewandten Seite konisch derart verbreitert, daß die in radialer Richtung innen liegende Stimfläche 13 des Befestigungsabschnitts 11 etwa halbiert wird.

Um einen Tampon zu pressen, wird zunächst der in der Regel aus einem gewickelten bandförmigen Faservlies bestehende Tamponrohling 20 zwischen den Stirnflächen 41 der Preßschneiden 40 zentriert. Sodann werden die Preßwerkzeuge 10 zugleich soweit linear verfahren, daß sich ein von den Stirnflächen 41 definierter Tamponkem 21 vorgegebenen Durchmessers ergibt.

Der Tamponkern 21 ist vergleichsweise hoch verdichtet und weist demzufolge eine hohe Knickfestigkeit auf. Aufgrund der bezüglich der Stirnflächen 41 in radialer Richtung zurückstehenden Stirnflächen 31 der Preßschultern 30 wird der Tamponrohling 20 in den Bereichen zwischen den Preßschneiden 40 geringer verformt. Dies hat zur Folge, daß verhältnismäßig weiche Längsrippen 22 gebildet werden, die eine grobe Faserstruktur aufweisen und demnach von einer hohen Absorptionsfähigkeit sind. In Fig. 1a ist zu erkennen, daß im geschlossenen Zustand der Preßwerkzeuge 10 die Anlageflächen 32 im Bereich des Übergangs zur Stirnfläche 31 unter Bildung einer annähernd linienförmigen Kontaktfläche an den jeweils benachbarten Preßschneiden 40 anliegen, wodurch verhindert wird, daß Faservlies zwischen die Preßwerkzeuge 10 gelangt.

Nach erfolgter Pressung des Tamponrohlings 20 zu einem Vorformling wird die Vorrichtung zum Pressen gelüftet, wozu die Preßwerkzeuge 10 ein Stück weit zurückgefahren werden. In dieser teilgeschlossenen Stellung der Preßwerkzeuge 10, die in Fig. 1b dargestellt ist, läßt sich der Vorformling beispielsweise mittels eines Stößels ausstoßen und in ein nachgeschaltetes Formwerkzeug einführen, in der er seine Endform erhält. Das nachgeschaltete Formwerkzeug ist dabei regelmäßig als sich konisch verjüngende Hülse ausgebildet, in die der Vorformling gegebenenfalls über eine Zwischenstation mit einem Stößel größerer Stimfläche einführbar ist und auf seinen endgültigen Durchmesser verformt wird, wobei die Verformung dann vornehmlich im Bereich der Längsrippen 22 und unter weitgehender Beibehaltung der Faserstruktur stattfindet.

Die zuvor beschriebene Vorrichtung zum Pressen eines Tampons ermöglicht bei der Verwendung von Preßwerkzeugen 10 eines einziges Typs und damit einer einfachen Verfahrenssteuerung eine variable Anpassung auf unterschiedliche Tampondurchmesser, ohne daß es aufwendiger Umrüstarbeiten bedarf. Von Vorteil ist überdies, daß sich hierbei auch das Materialverhältnis von gepreßtem Tamponkem 21 und den vergleichsweise weichen Längsrippen 22 einfach variieren läßt. Weiterhin ist durch die lösbare Verbindung von Preßschultern 30 und Preßschneiden 40 sichergestellt, daß die Preßschultern 30 und/oder die Preßschneiden 40 austauschbar sind, um etwa in ihrer Form oder ihren geometrischen Abmessungen auf die jeweils zu pressende Tampongröße angepaßt zu werden. Auf diese Weise lassen sich bei unterschiedlichen Tampondurchmessem Anlageflächen 32 ausgestalten, durch die vermieden wird, daß je nach Durchmesser der zu pressenden Tampons zwischen den jeweiligen Preßwerkzeugen 10 im geschlossenen Zustand durchgängige Öffnungen entstehen, in die Faservlies eindringen und sich ablagem kann. Nicht zuletzt wird damit auch einem geringeren Verschleiß und einer hierdurch bedingten einfacheren Wartung und höheren Lebensdauer Rechnung getragen.

### Bezugszeichenliste

- 10: Preßwerkzeug
- 11: Befestigungsabschnitt
- 12: Bohrung
- 13: Stirnfläche
- 20: Tamponrohling
- 21: Tamponkem
- 22: Längsrippe
- 30: Preßschulter
- 31: Stirnfläche
- 32: Anlagefläche
- 33: Klemmfläche
- 34: Ausnehmung
- 40: Preßschneide
- 41: Stimfläche
- 42: Seitenfläche
- 50: Schraubenverbindung
- 60: Schieberführung
- 70: Bohrung
- 71: Innengewinde
- 80: Schraube
- 81: Schraubenkopf
- 90: Langloch

## Patentansprüche

1. Vorrichtung zum Pressen eines Tampons, insbesondere für die Frauenhygiene, mit über den Umfang eines zu pressenden Tamponrohlings (20) gleichmäßig verteilt angeordneten Preßwerkzeugen (10), die radial zur Längsrichtung des Tamponrohlings (20) verfahrbar sind und mit denen die gesamte Umfangsfläche des Tamponrohlings (20) mit radialem Druck beaufschlagbar ist, wobei die Preßwerkzeuge (10) jeweils eine Preßfläche aufweisen, die sich aus der Stimfläche (31) einer Preßschulter (30) zur Bildung von verhältnismäßig weichen Längsrippen hoher Absorptionsfähigkeit und einer im Vergleich zur Preßschulter (30) in radialer Richtung vorstehenden Preßschneide (40) zur Bildung eines vergleichsweise verdichteten Tamponkems hoher Knickfestigkeit zusammensetzt,
**dadurch gekennzeichnet,**
**daß** die Preßschulter (30) und die Preßschneide (40) eines jeden Preßwerkzeugs (10) relativ zueinander verstellbar sind.

2. Vorrichtung nach Anspruch 1, **dadurch gekennzeichnet, daß** die Preßschulter (30) an einer Seitenfläche (42) der Preßschneide (40) in radialer Richtung verschiebbar angeordnet ist.

3. Vorrichtung nach Anspruch 2, **dadurch gekennzeichnet, daß** die Seitenfläche (42) der Preßschneide (40) als Führungsbahn für die Preßschulter (30) ausgebildet ist, wobei die Führungsbahn vorzugsweise eine flache Querschnittsform aufweist.

4. Vorrichtung nach einem der Ansprüche 1 bis 3, **dadurch gekennzeichnet, daß** die Preßschulter (30) und/oder die Preßschneide (40) mit wenigstens einem Langloch (90) für eine Schraubenverbindung (80) versehen ist, mit der die Preßschulter (30) und die Preßschneide (40) relativ zueinander verstellbar und lösbar miteinander verbindbar sind.

5. Vorrichtung nach Anspruch 4, **dadurch gekennzeichnet, daß** wenigstens zwei unterschiedlich orientierte Langlöcher (90) vorgesehen sind.

6. Vorrichtung nach einem der Ansprüche 2 bis 5, **dadurch gekennzeichnet, daß** zumindest ein Teil der der Preßschneide (40) abgewandten Seitenfläche der Preßschulter (30) eines Preßwerkzeugs (10) als Anlagefläche (32) für das benachbart angrenzende Preßwerkzeug (10) ausgebildet ist.

7. Vorrichtung nach Anspruch 6, **dadurch gekennzeichnet, daß** die Anlagefläche (32) eine annähernd linienförmige Kontaktfläche bildet.

8. Vorrichtung nach einem der Ansprüche 1 bis 7, **dadurch gekennzeichnet, daß** die Stirnfläche (31) der Preßschulter (30) im wesentlichen konkav geformt ist.

9. Vorrichtung nach einem der vorherigen Ansprüche, **dadurch gekennzeichnet, daß** die Preßwerkzeuge (10) identisch gestaltet und in radialer Richtung verfahrbar sind.

## Claims

1. Apparatus for pressing a tampon, particularly for feminine hygiene, having press dies (10) arranged in a manner uniformly distributed over the circumference of a tampon blank (20) that is to be pressed, it being possible for said press dies (10) to be moved radially in relation to the longitudinal direction of the tampon blank (20) and for said press dies (10) to exert radial pressure on the entire circumferential area of the tampon blank (20), each of the press dies (10) having a pressing surface which is composed of the end face (31) of a pressing shoulder (30) for forming relatively soft longitudinal ribs with a high absorption capability, and of a press cutter (40), which projects beyond the pressing shoulder (30) in the radial direction, for forming a comparatively compressed tampon core with high buckling strength, **characterized in that** the pressing shoulder (30) and the press cutter (40) of any one press die (10) can be adjusted relative to one another.

2. Apparatus according to Claim 1, **characterized in that** the pressing shoulder (30) is arranged on a side face (42) of the press cutter (40) such that it can be displaced in the radial direction.

3. Apparatus according co Claim 2, **characterized in that** the side face (42) of the press cutter (40) is designed as a guideway for the pressing shoulder (30), the guideway preferably having a flat cross-sectional shape.

4. Apparatus according to any of Claims 1 to 3, **characterized in that** the pressing shoulder (30) and/or the press cutter (40) is/are provided with at least one longitudinal hole (90) for a screw connection (80), by means of which the pressing shoulder (30) and the press cutter (40) can be adjusted relative to one another and can be releasably connected to one another.

5. Apparatus according to Claim 4, **characterized in that** at least two longitudinal holes (90) of different orientation are provided.

6. Apparatus according to any of Claims 2 to 5, **characterized in that** at least part of that side face of the pressing shoulder (30) of a press die (10) that faces away from the press cutter (40) is designed as a bearing surface (32) for the adjoining press die (10).

7. Apparatus according to Claim 6, **characterized in that** the bearing surface (32) forms an approximately linear contact surface.

8. Apparatus according to any of Claims 1 to 7, **characterized in that** the end face (31) of the pressing shoulder (30) is of essentially concave shape.

9. Apparatus according to any of the preceding claims, **characterized in that** the press dies (10) are of identical configuration and can be moved in the radial direction.

## Revendications

1. Dispositif de compression d'un tampon, notamment pour l'hygiène des femmes, comprenant des outils de compression (10) régulièrement répartis sur la circonférence d'une ébauche de tampon (20) à comprimer, les outils de compression étant déplaçables radialement par rapport à la direction longitudinale de l'ébauche de tampon (20) et servant à appliquer une pression radiale sur l'intégralité de la surface circonférentielle de l'ébauche de tampon (20), chacun des outils de compression (10) comprenant une surface de compression, qui est composée de la face (31) d'une épaule de compression (30) pour former des nervures longitudinales et relativement souples et présentant une haute absorption et d'une lame de compression (40), qui fait saillie dans une direction radiale par rapport à l'épaule de compression (30), pour former un noyau de tampon, qui est comparativement comprimé et présente une haute résistance au flambage,
**caractérisé en ce**
**que** l'épaule de compression (30) et la lame de compression (40) de chaque outil de compression (10) sont déplaçables l'une par rapport à l'autre.

2. Dispositif selon la revendication 1, **caractérisé en ce que** l'épaule de compression (30) est disposée de manière déplaçable dans une direction radiale sur une face latérale (42) de la lame de compression (40)

3. Dispositif selon la revendication 2, **caractérisé en ce que** la face latérale (42) de la lame de compression (40) forme une voie de guidage pour l'épaule de compression (30), la voie de guidage comprenant de préférence une forme de section transversale plane.

4. Dispositif selon l'une des revendications 1 à 3, **caractérisé en ce que** l'épaule de compression (30) et/ou la lame de compression (40) sont munies d'au moins un trou oblong (90) pour un vissage (80), par moyen duquel on peut mutuellement relier l'épaule de compression (30) et la lame de compression (40) de manière déplaçable l'une à l'autre et amovible.

5. Dispositif selon la revendication 4, **caractérisé en ce qu'**on prévoit au moins deux trous oblongs (90) ayant une orientation différente.

6. Dispositif selon l'une des revendications 2 à 5, **caractérisé en ce qu'**au moins une partie de la face latérale de l'épaule de compression (30) d'un outil de compression (10), qui est opposée à la lame de compression (40), forme une face d'appui (32) pour l'outil de compression (10) adjacent.

7. Dispositif selon la revendication 6, **caractérisé en ce que** la face d'appui (32) forme une face de contact approximativement linéaire.

8. Dispositif selon l'une des revendications 1 à 7, **caractérisé en ce que** la face (31) de l'épaule de compression (30) a une forme essentiellement concave.

9. Dispositif selon l'une des revendications précédentes, **caractérisé en ce que** les outils de compression (10) ont une forme identique et sont déplaçables dans une direction radiale.
